Europäisches Patentamt
European Patent Office
Office européen des brevets

(19) (11) Veröffentlichungsnummer: **0 133 950 A1**

# (12) EUROPÄISCHE PATENTANMELDUNG

(21) Anmeldenummer: **84108665.5**

(22) Anmeldetag: **23.07.84**

(51) Int. Cl.⁴: **C 07 D 405/06**, A 01 N 43/40 // (C07D405/06, 211/22, 317/28)

(30) Priorität: **04.08.83 DE 3328151**

(43) Veröffentlichungstag der Anmeldung: **13.03.85 Patentblatt 85/11**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Krämer, Wolfgang, Dr., Am Eckbusch 39/45, D-5600 Wuppertal 1 (DE)**
Erfinder: **Weissmüller, Joachim, Dr., Sillerstrasse 49, D-5600 Wuppertal 11 (DE)**
Erfinder: **Berg, Dieter, Dr., Gellertweg 27, D-5600 Wuppertal 1 (DE)**
Erfinder: **Reinecke, Paul, Dr., Lessingstrasse 11, D-5090 Leverkusen 3 (DE)**
Erfinder: **Hänssler, Gerd, Dr., Heymannstrasse 40, D-5090 Leverkusen (DE)**
Erfinder: **Brandes, Wilhelm, Dr., Eichendorffstrasse 3, D-5653 Leichlingen (DE)**

## (54) Substituierte 4-Piperidinomethyl-1,3-dioxolane.

(57) Substituierte 4-Piperidinomethyl-1,3-dioxolane der Formel (I)

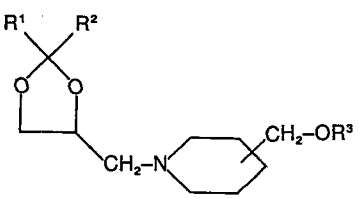

(I)

in welcher

$R^1$ für Tetrahydronaphthyl, Decahydronaphthyl oder für gegebenenfalls substituiertes Naphthyl, für gegebenenfalls substituiertes Cycloalkyl und Cycloalkenyl, für gegebenenfalls substituiertes Phenyl oder für durch – jeweils gegebenenfalls substituiertes Phenyl, Phenoxy, Phenylthio, Cyclohexyl, Cyclohexyloxy oder Cyclohexylthio substituiertes Alkyl steht,

$R^2$ für Wasserstoff oder Methyl steht und

$R^3$ für Wasserstoff oder einen Acylrest steht,

und deren pflanzenverträgliche Säureadditionssalze und Metallsalzkomplexe sowie ihre Verwendung als Schädlingsbekämpfungsmittel, vor allem als Fungizide.

Die neuen substituierten 4-Piperidinomethyl-1,3-dioxolane können hergestellt werden, wenn man geeignete in 4-Stellung substituierte Dioxolane mit Hydroxymethylpiperidinen umsetzt und gegebenenfalls die dabei erhaltenen, erfindungsgemäßen 4-[Hydroxymethylpiperidinomethyl]-1,3-dioxolane an der Hydroxylgruppe mit geeigneten Acylierungsmitteln, z.B. Acylhalogeniden, Acylanhydriden oder Isocyanaten, acyliert.

EP 0 133 950 A1

BAYER AKTIENGESELLSCHAFT 5090 Leverkusen, Bayerwerk

Konzernverwaltung RP
Patentabteilung

Bas/AB

Ia

# Substituierte 4-Piperidinomethyl-1,3-dioxolane

Die Erfindung betrifft neue substituierte 4-Piperidinomethyl-1,3-dioxolane, ein Verfahren zu ihrer Herstellung und ihre Verwendung als Schädlingsbekämpfungsmittel.

Es ist bereits bekannt, daß bestimmte 1,3-Dioxolane, wie beispielsweise das 2-[1-(4-Chlorphenoxy)-2-methyl-prop-2-yl]-4-ethyl-2-(1,2,4-triazol-1-ylmethyl)-1,3-dioxolan Hydrochlorid (vgl. DE-OS 3 104 311 bzw. EP 57 864),oder auch organische Schwefelverbindungen, wie beispielsweise das Zinkethylen-1,2-bis-(dithiocarbamat) (vgl. R.Wegler, 'Chemie der Pflanzenschutz- und Schädlingsbekämpfungsmittel', Springer Verlag, Berlin, Heidelberg, New York 1970, Band 2, S. 65 f) fungizide Eigenschaften besitzen.

Die Wirkung dieser Verbindungen ist jedoch unter bestimmten Umständen, insbesondere bei niedrigen Aufwandmengen und -konzentrationen, in einigen Anwendungsbereichen nicht immer völlig zufriedenstellend.
Es wurden neue substituierte 4-Piperidinomethyl-1,3-dioxolane der allgemeinen Formel (I)

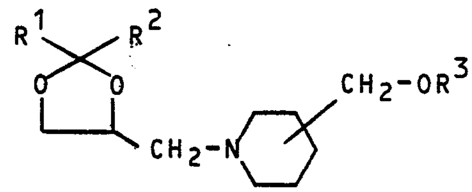

(I)

in welcher

$R^1$ für Tetrahydronaphthyl, Decahydronaphthyl oder für gegebenenfalls substituiertes Naphthyl, für gegebenenfalls substituiertes Cycloalkyl und Cycloalkenyl, für gegebenenfalls substituiertes Phenyl oder für durch - jeweils gegebenenfalls substituiertes - Phenyl, Phenoxy, Phenylthio, Cyclohexyl, Cyclohexyloxy oder Cyclohexylthio substituiertes Alkyl steht,

$R^2$ für Wasserstoff oder Methyl steht und

$R^3$ für Wasserstoff oder einen Acylrest steht,

sowie deren pflanzenverträgliche Säureadditionssalze und Metallsalzkomplexe gefunden.

Die Verbindungen der Formel (I) können als geometrische und/oder optische Isomere oder Isomerengemische unterschiedlicher Zusammensetzung anfallen. Sowohl die reinen Isomeren als auch die Isomerengemische werden erfindungsgemäß beansprucht.

Weiterhin wurde gefunden, daß man die neuen substituierten 4-Piperidinomethyl-1,3-dioxolane der allgemeinen Formel (I)

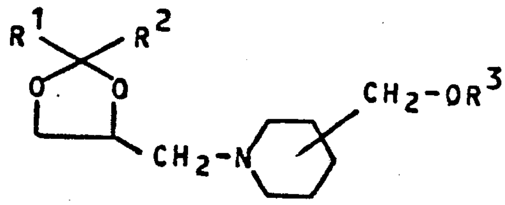

(I)

in welcher

$R^1$ für Tetrahydronaphthyl, Decahydronaphthyl oder für gegebenenfalls substituiertes Naphthyl, für gegebenenfalls substituiertes Cycloalkyl und Cycloalkenyl, für gegebenenfalls substituiertes Phenyl oder für durch - jeweils gegebenenfalls substituiertes — Phenyl, Phenoxy, Phenylthio, Cyclohexyl, Cyclohexyloxy oder Cyclohexylthio substituiertes Alkyl steht,

$R^2$ für Wasserstoff oder Methyl steht und

$R^3$ für Wasserstoff oder einen Acylrest steht,

sowie deren pflanzenverträgliche Säureadditionssalze und Metallsalzkomplexe erhält, wenn man in 4-Stellung substituierte Dioxolane der allgemeinen Formel (II)

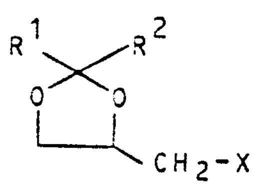

(II)

in welcher

$R^1$ und $R^2$ die oben angegebenen Bedeutung haben und

X für Halogen oder für gegebenenfalls substituiertes Alkylsulfonyloxy bzw. Arylsulfonyloxy steht,

mit Hydroxymethylpiperidinen der allgemeinen Formel (III)

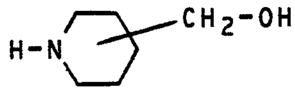

(III)

gegebenenfalls in Gegenwart eines Verdünnungsmittels, gegebenenfalls in Gegenwart eines Katalysators und gegebenenfalls in Gegenwart eines Säurebindemittels umsetzt und gegebenenfalls in einer 2. Stufe die so erhaltenen, erfindungsgemä 4-(Hydroxymethylpiperidinomethyl)-1,3-dioxolane der allgemeinen Formel (Ia),

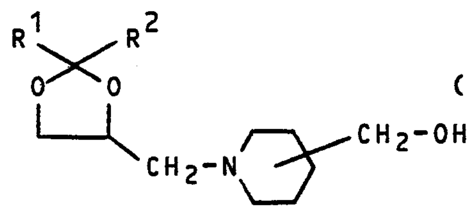

(Ia)

in welcher

$R^1$ und $R^2$ die oben angegebenen Bedeutungen haben,

in prinzipiell bekannter Weise gegebenenfalls in Gegenwart eines Verdünnungsmittels und gegebenenfalls in Gegenwart eines Säurebindemittels mit einem Acylierungsmittel am Sauerstoff der Hydroxymethylgruppe acyliert und gegebenenfalls anschließend eine Säure oder ein Metallsalz addiert.

Außerdem ist es möglich, die erfindungsgemäßen 4-Piperidinomethyl-1,3-dioxolane der Formel (I) nach allgemein üblichen Methoden am Stickstoff zu quarternisieren zu den entsprechenden tetrasubstituierten Ammoniumsalzen.

Schließlich wurde gefunden, daß die neuen substituierten 4-Piperidinomethyl-1,3-dioxolane der Formel (I) fungizide Eigenschaften besitzen.

Dabei zeigen die erfindungsgemäßen Verbindungen der Formel (I) überraschenderweise eine höhere fungizide Wirksamkeit als die aus dem Stand der Technik bekannten Verbindungen 2-[1-(4-Chlorphenoxy)-2-methyl-prop-2-yl]-4-ethyl-2-(1,2,4-triazol-1-yl-methyl)-1,3-dioxolan Hydrochlorid oder Zink-ethylen-1,2-bis-(dithiocarbamat), welches chemisch und/oder wirkungsmäßig naheliegende Verbindungen sind. Die erfindungsgemäßen Verbindungen stellen somit eine Bereicherung der Technik dar.

Die erfindungsgemäßen substituierten 4-Piperidinomethyl-1,3-dioxolane sind durch die Formel (I) allgemein definiert.

Bevorzugt sind Verbindungen der Formel (I), bei denen

$R^1$ für Tetrahydronaphthyl, Decahydronaphthyl oder für gegegebenenfalls einfach bis siebenfach, gleich oder verschieden substituiertes Naphthyl steht, wobei als Substituenten infrage kommen : Hydroxy, Halogen, jeweils geradkettiges oder verzweigtes Alkyl,Alkoxy, Alkenyloxy, Alkinyloxy oder Alkanoyloxy mit jeweils bis zu 4 Kohlenstoffatomen in den Alkylteilen oder weiterhin für jeweils gegebenenfalls einfach oder mehrfach, gleich oder verschieden durch geradkettiges oder verzweigtes Alkyl mit bis zu 4 Kohlenstoffatomen substituiertes

Cycloalkyl oder Cycloalkenyl mit jeweils 3 bis 7 Kohlenstoffatomen steht, für gegebenenfalls einfach oder mehrfach, gleich oder verschieden substituiertes Phenyl steht, oder für im Phenylkern gegebenenfalls einfach oder mehrfach, gleich oder verschieden substituiertes Phenylalkyl, Phenoxyalkyl oder Phenylthioalkyl mit jeweils bis zu 6 Kohlenstoffatomen im geradkettigen oder verzweigten Alkylteil steht, wobei als Phenylsubstituenten jeweils infrage kommen : Hydroxy, Halogen, Cyano, Nitro, jeweils geradkettiges oder verzweigtes Alkyl, Alkoxy, Alkenyloxy, Alkinyloxy und Alkylthio mit jeweils bis zu 4 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, Cycloalkyl mit 5 bis 7 Kohlenstoffatomen, geradkettiges oder verzweigtes Alkoxycarbonyl bzw. Alkanoyloxy mit jeweils bis zu 4 Kohlenstoffatomen im Alkylteil, gegebenenfalls durch Halogen, insbesondere Fluor oder Chlor, oder durch geradkettiges oder verzweigtes Alkyl mit bis zu 4 Kohlenstoffatomen substituiertes Phenyl oder Phenoxy, sowie der Rest R-O-N=CH-, wobei R jeweils für geradkettiges oder verzweigtes Alkyl, Alkenyl oder Alkinyl mit bis zu 4 Kohlenstoffatomen steht; außerdem für jeweils im Cyclohexylteil gegebenenfalls einfach oder mehrfach, gleich oder verschieden durch geradkettiges oder verzweigtes Alkyl mit bis zu 4 Kohlenstoffatomen substituiertes Cyclohexylalkyl, Cyclohexyloxyalkyl oder Cyclohexylthioalkyl mit jeweils bis zu 6 Kohlenstoffatomen in den geradkettigen oder verzweigten Alkylteilen steht,

$R^2$ für Wasserstoff oder Methyl steht und

$R^3$ für Wasserstoff oder für einen Rest der Formel

$R^4-\underset{\underset{O}{\parallel}}{C}-$ steht,

worin

$R^4$ für geradkettiges oder verzweigtes Alkyl, Alkoxy oder Alkylamino bzw. Dialkylamino mit jeweils 1 bis 6 Kohlenstoffatomen in den einzelnen Alkylteilen, für geradkettiges oder verzweigtes Alkoxyalkyl mit jeweils 1 bis 6 Kohlenstoffatomen in beiden Alkylteilen, für geradkettiges oder verzweigtes Halogenalkyl mit bis zu 6 Kohlenstoffatomen und bis zu 5 gleichen oder verschiedenen Halogenatomen oder für den Furylrest steht.

Besonders bevorzugt sind Verbindungen der allgemeinen Formel (I), bei denen

$R^1$ für Tetrahydronaphthyl, Decahydronaphthyl oder für gegebenenfalls einfach bis dreifach, gleich oder verschieden substituiertes Naphthyl steht, wobei als Substituenten infrage kommen : Fluor, Chlor, Brom, Iod, Hydroxy, Methyl, Ethyl, Methoxy, Ethoxy, Allyloxy, Propargyloxy und Acetyloxy, weiterhin für gegebenenfalls einfach bis dreifach, gleich oder verschieden durch Methyl oder Ethyl substituiertes Cycloalkyl oder Cycloalkenyl mit 5 bis 7 Kohlenstoffatomen, für gegebenenfalls einfach bis dreifach, gleich oder verschieden substituiertes Phenyl oder für einen im Phenylkern gegebenenfalls einfach bis dreifach, gleich oder verschieden substituierten Rest der Formel

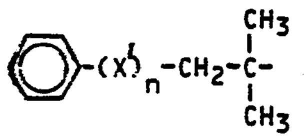 ; 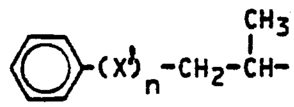 ; 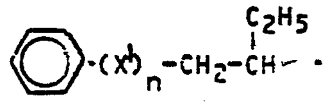

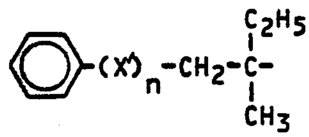 oder 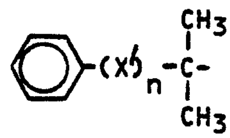 steht,

wobei als Phenylsubstituenten jeweils infrage kommen : Hydroxy, Fluor, Chlor, Brom, Iod, Cyano, Nitro, Methyl, Methoxy, Methylthio, Ethyl, Ethoxy, Ethylthio, n- und i-Propyl, Isopropyloxy, n-, i-, s- und t-Butyl, Allyloxy, Propargyloxy, Trifluormethyl, Trifluormethoxy, Trifluormethylthio, Cyclohexyl, Methoxycarbonyl, Ethoxycarbonyl, Acetyloxy, gegebenenfalls durch Fluor, Chlor oder Methyl substituiertes Phenyl oder Phenoxy, sowie der Rest R-O-N=CH-, wobei R jeweils für Methyl, Ethyl, n- und i-Propyl, n-, i-, s- und t-Butyl, Allyl und Propargyl steht; außerdem für einen im Cyclohexylteil gegebenenfalls einfach bis dreifach, gleich oder verschieden durch Methyl, Ethyl oder Isopropyl substituierten Rest der Formel

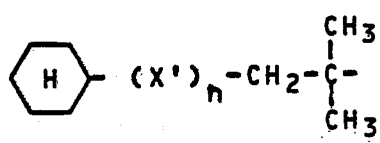 ; 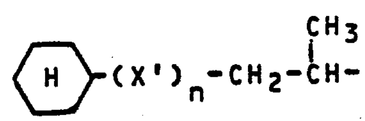 ;

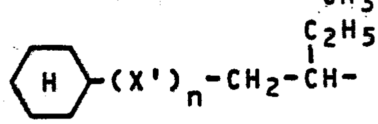 ; 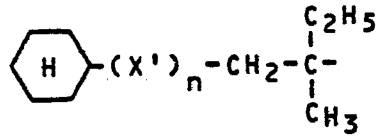 oder

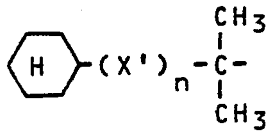 steht,

wobei in allen oben formelmäßig dargestellten Resten X' jeweils für Sauerstoff oder Schwefel und n für 0 oder 1 steht,

$R^2$ für Methyl steht und

$R^3$ für Wasserstoff oder für einen Rest der Formel 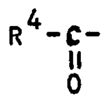 steht, wobei

$R^4$ für Methyl, Ethyl, n- und i-Propyl, Methoxymethyl, Chlormethyl, Dichlormethyl, Trichlormethyl, Methoxy, Ethoxy, Methylamino, Ethylamino, Dimethylamino, Diethylamino oder für den Furylrest steht.

Im einzelnen seien außer den bei den Herstellungsbeispielen genannten Verbindungen die folgenden Verbindungen der allgemeinen Formel (I) genannt :

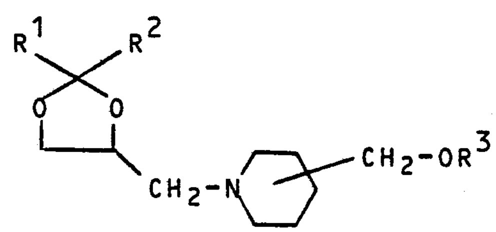

(I)

| $R^1$ | $R^2$ | 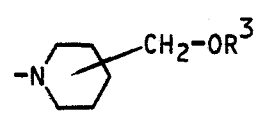 |
|---|---|---|
| 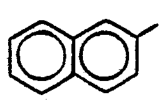 | $CH_3$ | 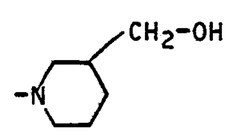 |
| 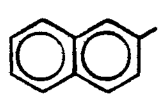 | $CH_3$ | 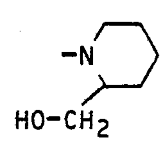 |

(Fortsetzung)

| $R^1$ | $R^2$ | 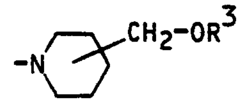 |
|---|---|---|
| 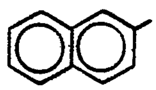 | $CH_3$ | 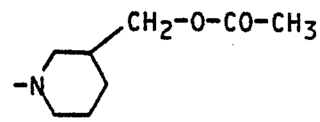 |
| 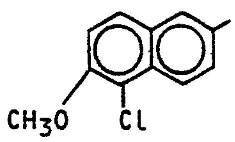 | $CH_3$ | 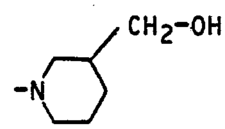 |
| 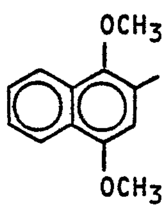 | $CH_3$ | 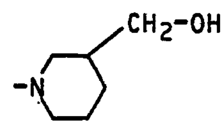 |
| 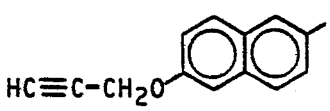 | $CH_3$ | 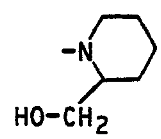 |
| 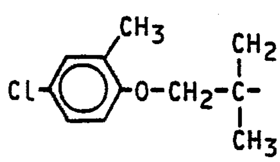 | $CH_3$ | 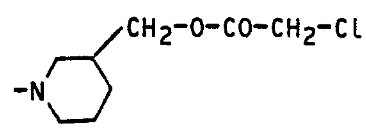 |
| 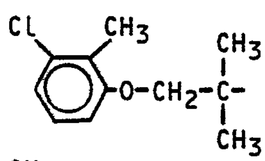 | $CH_3$ | 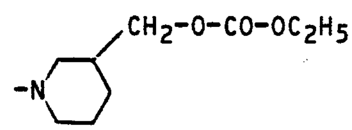 |
| 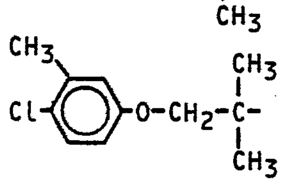 | $CH_3$ | 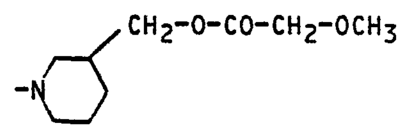 |
| 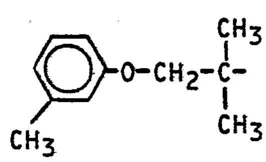 | $CH_3$ | 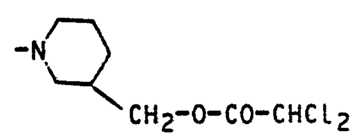 |

(Fortsetzung)

| $R^1$ | $R^2$ | 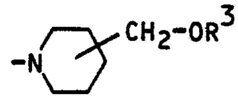 |
|---|---|---|
| 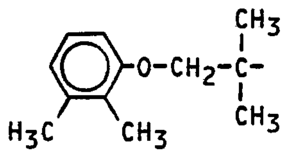 | $CH_3$ | 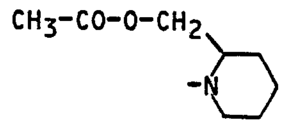 |
| 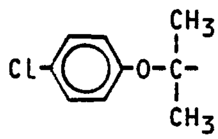 | $CH_3$ | 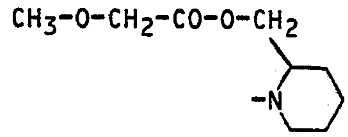 |
| 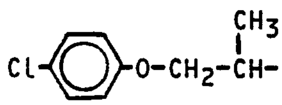 | $CH_3$ | 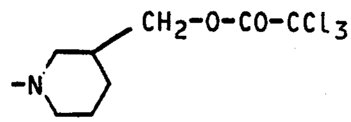 |
| 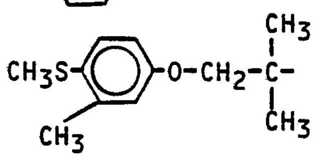 | $CH_3$ | 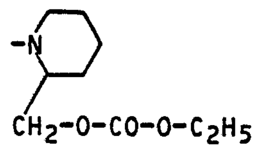 |
| 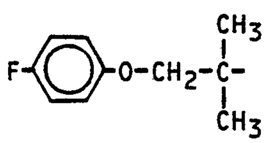 | $CH_3$ | 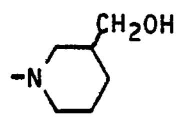 |
| 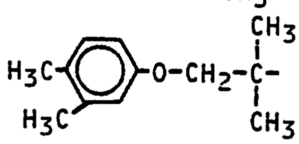 | $CH_3$ | 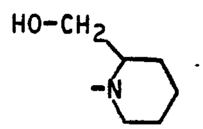 |
| 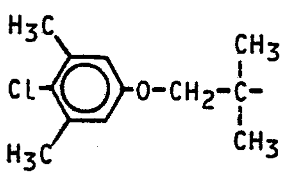 | $CH_3$ | 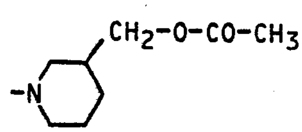 |
| 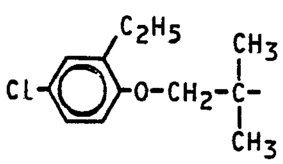 | $CH_3$ | 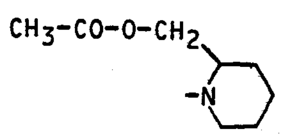 |
| 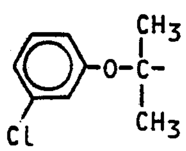 | $CH_3$ | 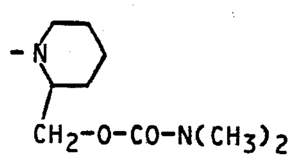 |

(Fortsetzung)

| $R^1$ | $R^2$ | 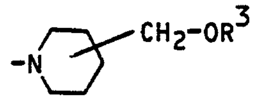 |
|---|---|---|
| 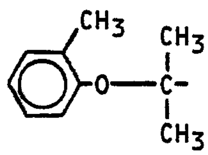 | $CH_3$ | 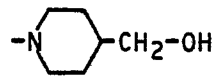 |
| 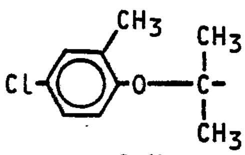 | $CH_3$ | 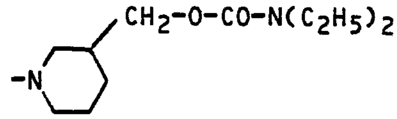 |
| 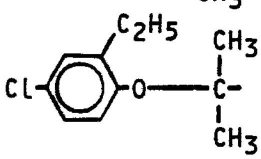 | $CH_3$ | 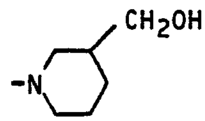 |
| 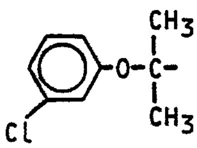 | $CH_3$ | 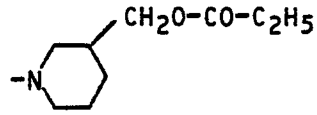 |
| 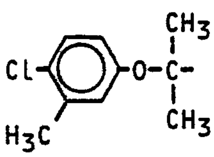 | $CH_3$ | 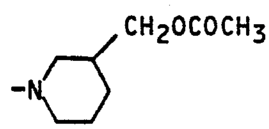 |
| 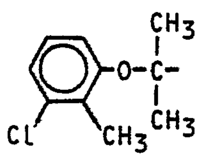 | $CH_3$ | 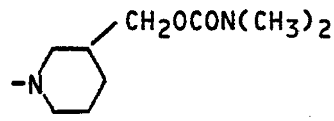 |
| 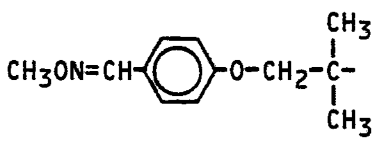 | $CH_3$ | 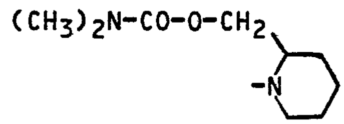 |
| 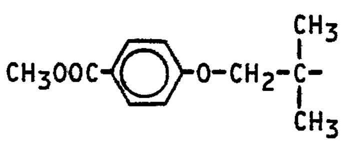 | $CH_3$ | 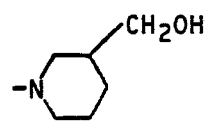 |

(Fortsetzung)

| $R^1$ | $R^2$ | 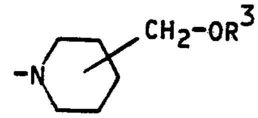 |
|---|---|---|
| 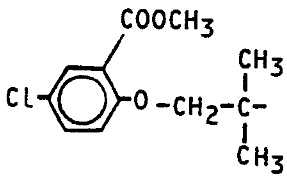 | $CH_3$ | 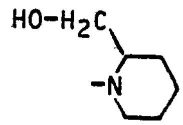 |
| 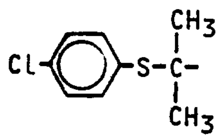 | $CH_3$ | 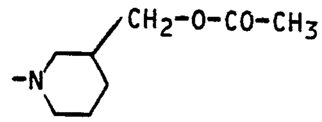 |
| 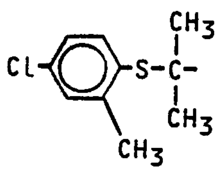 | $CH_3$ | 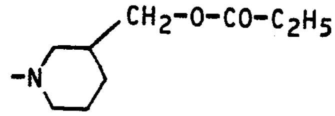 |
| 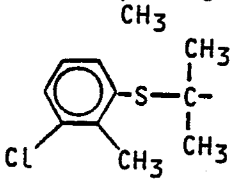 | $CH_3$ | 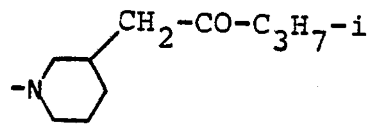 |
| 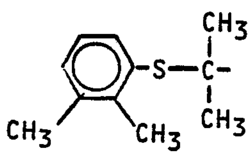 | $CH_3$ | 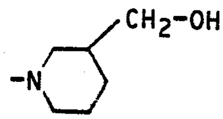 |
| 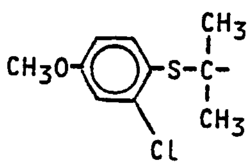 | $CH_3$ | 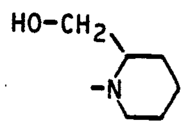 |
| 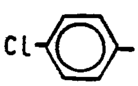 | $CH_3$ | 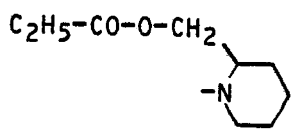 |
| 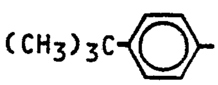 | $CH_3$ | 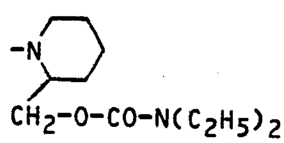 |
| 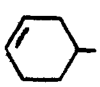 | $CH_3$ | 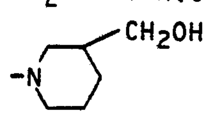 |
| 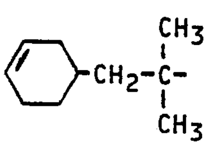 | $CH_3$ | 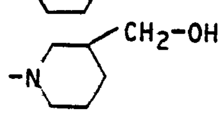 |

(Fortsetzung)

| $R^1$ | $R^2$ | 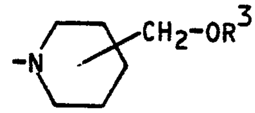 |
|---|---|---|
| 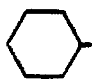 | $CH_3$ | 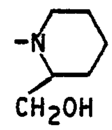 |
| 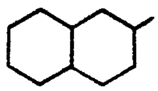 | $CH_3$ | 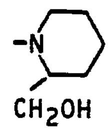 |
| 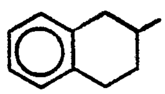 | $CH_3$ | 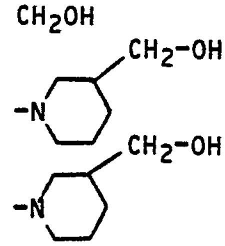 |
| 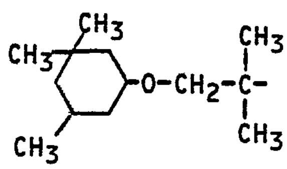 | $CH_3$ | |
| 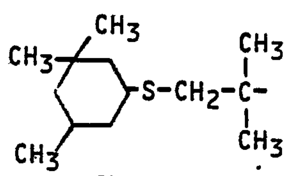 | $CH_3$ | 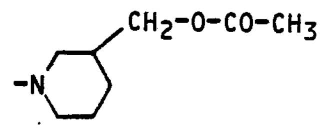 |
| 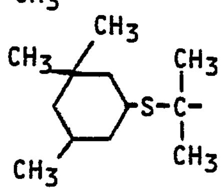 | $CH_3$ | 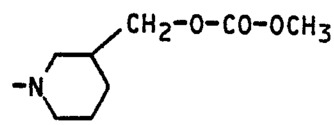 |
| 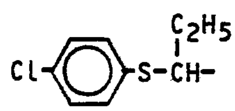 | $CH_3$ | 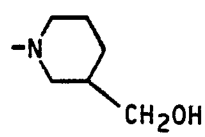 |
| 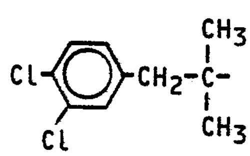 | $CH_3$ | 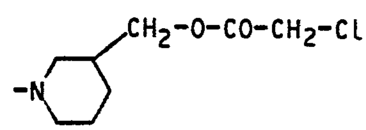 |

(Fortsetzung)

| $R^1$ | $R^2$ | 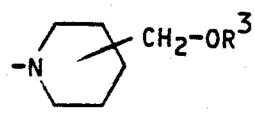 |
|---|---|---|
| 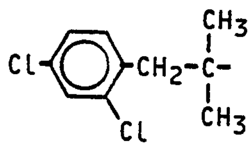 | $CH_3$ | 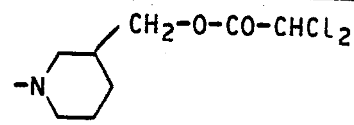 |
| 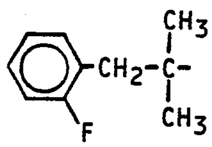 | $CH_3$ | 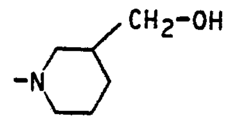 |
| 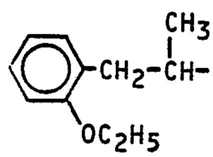 | $CH_3$ | 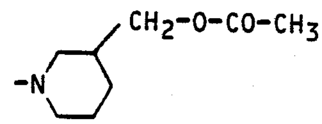 |
| 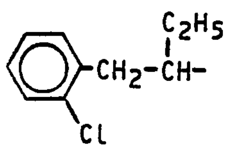 | $CH_3$ | 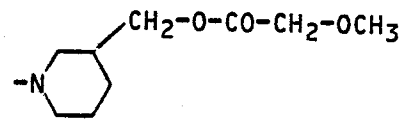 |
| 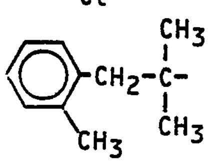 | $CH_3$ | 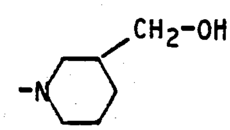 |
| 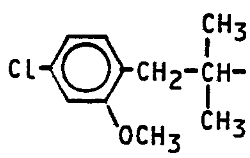 | $CH_3$ | 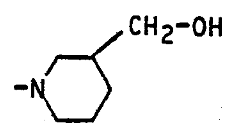 |

Verwendet man beispielsweise 4-Chlormethyl-2-[1-(4-chlorphenoxy)-2-methyl-prop-2-yl]-2-methyl-1,3-dioxolan und 3-Hydroxymethylpiperidin als Ausgangsstoffe sowie Acetylchlorid als Acylierungsmittel, so kann der Reaktionsablauf des erfindungsgemäßen Verfahrens durch das folgende Formelschema wiedergegeben werden :

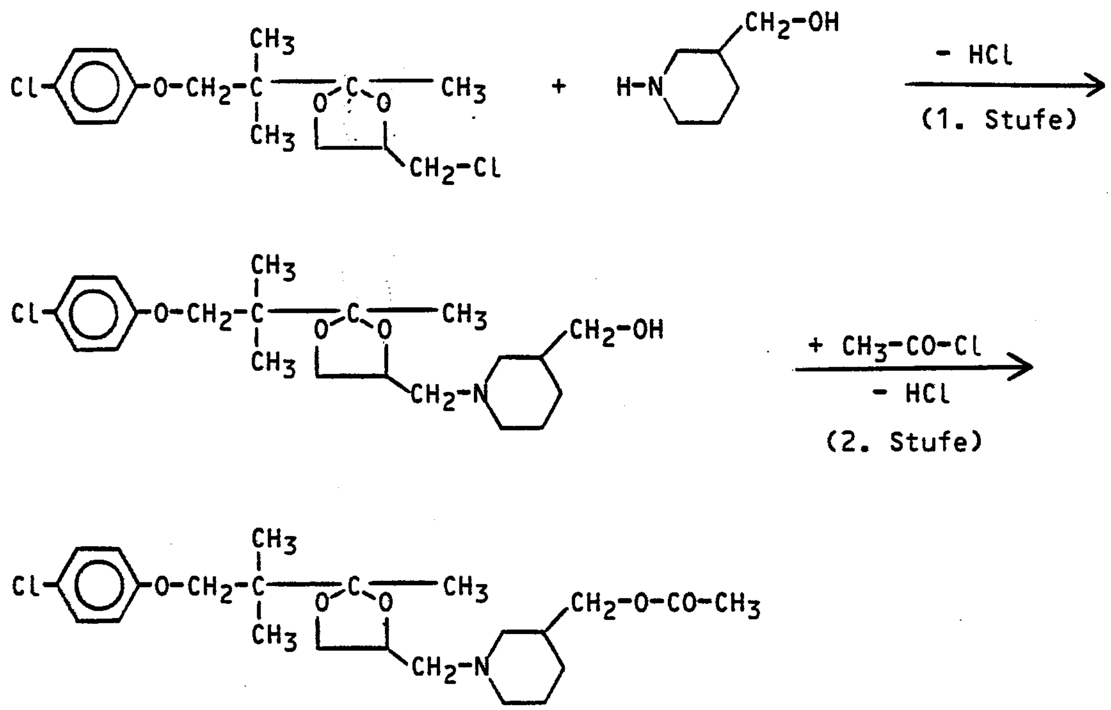


Die zur Durchführung des erfindungsgemäßen Verfahrens als Ausgangsstoffe benötigten in 4-Stellung substituierten Dioxolane sind durch die Formel (II) allgemein definiert. In dieser Formel haben $R^1$ und $R^2$ vorzugsweise diejenigen Bedeutungen, die bereits im Zusammenhang mit der Beschreibung der erfindungsgemäßen Stoffe der Formel (II) vorzugsweise für diese Reste genannt wurden. X steht vorzugsweise für Chlor, Brom, Methansulfonyloxy, p-Toluolsulfonyloxy oder Trifluormethansulfonyloxy.

Die in 4-Stellung substituierten Dioxolane der Formel (II) sind teilweise bekannt (vgl. z.B. Rec.Trav.Chim.Pays-Bas 91, 989-1001 (1972); Farm.Ed.Sci. 29, 167-174 (1974); J.med.Chem. 6 (1963), 3, 325-328) oder können nach den dort angegebenen Verfahren in analoger Weise erhalten werden, indem man z.B. Aldehyde oder Ketone der Formel (IV)

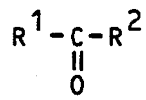 (IV)

in welcher
$R^1$ und $R^2$ die oben angegebene Bedeutungen haben,

in üblicher Weise mit substituierten 1,2-Diolen der Formel (V)

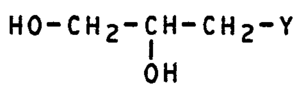 (V)

in welcher
Y für Halogen oder Hydroxy steht,

in Gegenwart eines inerten organischen Lösungsmittels, wie z.B. Toluol, und in Gegenwart eines Katalysators, wie z.B. p-Toluolsulfonsäure , bei Temperaturen zwischen 50°C und 120°C umsetzt;

und in den Fällen, wo Y für die Hydroxygruppe steht, die so erhaltenen 4-Hydroxymethyl-1,3-dioxolane der Formel (IIa)

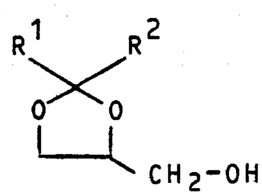 (IIa)

in welcher
$R^1$ und $R^2$ die oben angegebene Bedeutungen haben,

mit gegebenenfalls substituierten Alkyl- bzw. Arylsulfonylhalogeniden der Formel (VI),

$$Z\text{-}SO_2\text{-}Hal \qquad (VI)$$

in welcher
Hal für Halogen, vorzugsweise für Chlor steht und
Z für gegebenenfalls substituiertes Alkyl bzw. Aryl, vorzugsweise für Methyl, Trifluormethyl oder 4-Methylphenyl, steht,

gegebenenfalls in Gegenwart eines Verdünnungsmittels, wie beispielsweise Diethylether, und gegebenenfalls in Gegenwart eines Säurebindemittels, wie beispielsweise Pyridin oder Triethylamin, bei Temperaturen zwischen -20°C und +100°C umsetzt zu den Verbindungen der Formel (IIb),

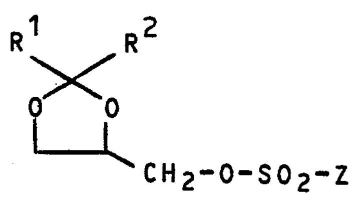

(IIb)

in welcher
$R^1$, $R^2$ und Z die oben angegebene Bedeutung haben.

Die Ketone der Formel (IV) sind bekannt (vgl. z.B. DE-OS 32 10 725 oder DE-OS 30 48 266) oder können nach bekannten Methoden in einfacher Weise erhalten werden.

Die substituierten 1,2-Diole der Formel (V) und die Sulfonylhalogenide der Formel (VI) sind allgemein bekannte Verbindungen der organischen Chemie.

Die außerdem für das erfindungsgemäße Verfahren als Ausgangsstoffe benötigten Hydroxymethylpiperidine sind durch die Formel (III) allgemein definiert. Bevorzugte Verbindungen der Formel (III) sind das 2-Hydroxymethylpiperidin und das 3-Hydroxymethylpiperidin. Die Hydroxymethylpiperidine der Formel (III) sind bekannt (vgl. z.B. Beilstein 'Handbuch der organischen Chemie', Band 21, II, Seite 8).

Für die gegebenenfalls durchzuführende 2. Stufe des erfindungsgemäßen Verfahrens (Acylierung) verwendet man als Acylierungsmittel vorzugsweise

a) Halogenide oder Anhydride der Formel (VII)

$$R^4\text{-CO-A} \qquad (VII)$$

in welcher

$R^4$ für Alkyl, insbesondere Methyl, Ethyl, n- und i-Propyl, für Alkoxy, insbesondere Methoxy oder Ethoxy, für Dialkylamino, insbesondere Dimethylamino, Diethylamino, für Alkoxyalkyl, insbesondere Methoxymethyl, für Halogenalkyl, insbesondere Chlormethyl, Dichlormethyl, Trichlormethyl oder den Furylrest steht und

A für Halogen, insbesondere Fluor, Chlor oder Brom oder für einen Rest der Formel $R^4$-CO-O- steht, wobei $R^4$ die oben angegebenen Bedeutungen hat,

oder

b) Isocyanate der Formel (VIII)

$$R^5-N=C=O \quad \text{(VIII)}$$

in welcher
$R^5$ für Alkyl, insbesondere für Methyl oder Ethyl steht.

Die Acylierungsmittel der Formeln (VII) und (VIII) sind allgemein bekannte Verbindungen der organischen Chemie.

Als Verdünnungsmittel kommen für die 1. Stufe des erfindungsgemäßen Verfahrens organische Lösungsmittel infrage. Hierzu gehören vorzugsweise aromatische Kohlenwasserstoffe, wie Benzol, Toluol oder Xylol; halogenierte Kohlenwasserstoffe, wie Tetrachlorkohlenstoff oder Chlorbenzol; Formamide, wie Dimethylformamid; Nitrile, wie Acetonitril oder Propionitril; Alkohole, wie Propanol oder Butanol; Amine, wie Triethylamin oder Piperidin; sowie die hochpolaren Lösungsmittel Dimethylsulfoxid oder Hexamethylphosphorsäuretriamid.

Das erfindungsgemäße Verfahren (1. Stufe) wird gegebenenfalls in Gegenwart einer Base als Säurebindemittel durchgeführt. Hierbei können alle üblichen organischen und insbesondere anorganischen Basen eingesetzt werden. Hierzu gehören vorzugsweise Alkalimetallhydroxide oder -Carbonate, wie beispielsweise Natriumhydroxid, Natriumcarbonat oder Kaliumcarbonat; ferner Triethylamin und Pyridin.

Ebenfalls möglich ist die Verwendung eines entsprechenden Ueberschusses an Hydroxymethylpiperidin der Formel (III) als Säurebindemittel.

Die 1. Stufe des erfindungsgemäßen Verfahrens wird gegebenenfalls in Gegenwart eines Katalysators durchgeführt. Vorzugsweise verwendet man Alkalimetalljodide, wie z.B. Kaliumjodid.

Die Reaktionstemperaturen können bei dem erfindungsgemäßen Verfahren (1. Stufe) in einem größeren Bereich variiert werden. Im allgemeinen arbeitet man zwischen 50°C und 250°C, vorzugsweise zwischen 80°C und 200°C.

Das erfindungsgemäße Verfahren (1. Stufe) kann bei Normaldruck, aber auch bei erhöhtem Druck durchgeführt werden. Im allgemeinen arbeitet man bei Drucken zwischen etwa 1,5 atü und 5 atü, vorzugsweise zwischen 1,5 atü und 3 atü.

Bei der Durchführung des erfindungsgemäßen Verfahrens (1. Stufe) setzt man auf 1 Mol substituiertes Dioxolan der Formel (II) vorzugsweise 1 bis 3 Mol Hydroxymethylpiperidin der Formel (III) und 1 bis 3 Mol Base ein.

Die Aufarbeitung und Isolierung der 4-(Hydroxymethylpiperidinomethyl)-1,3-dioxolane der Formel (Ia) erfolgt nach üblichen Methoden.

Als Verdünnungsmittel für die gegebenenfalls durchzuführende 2. Stufe des erfindungsgemäßen Verfahrens (Acylierung) kommen ebenfalls organische Lösungsmittel infrage. Hierzu gehören vorzugsweise Kohlenwasserstoffe, wie Benzol, Toluol, Xylol oder Petrolether; halogenierte Kohlenwasserstoffe, wie Methylenchlorid , Chloroform oder

Tetrachlorkohlenstoff, Nitrile, wie Acetonitril oder Propionitril oder Ester, wie Essigsäureethylester.

Die 2. Stufe des erfindungsgemäßen Verfahrens (Acylierung) wird gegebenenfalls in Gegenwart eines Säurebindemittels durchgeführt. Als solche können alle üblichen organischen Basen verwendet werden. Vorzugsweise verwendet man tertiäre Amine, wie beispielsweise Triethylamin, N,N-Dimethylanilin, Dimethylaminopyridin oder Pyridin. Die Acylierung kann auch ohne Säurebindemittel durchgeführt werden.

Die Reaktionstemperaturen können bei der 2. Stufe des erfindungsgemäßen Verfahrens (Acylierung) in einem größeren Bereich variiert werden. Im allgemeinen arbeitet man zwischen -20°C und +100°C, vorzugsweise zwischen 0°C und 80°C.

Die 2. Stufe des erfindungsgemäßen Verfahrens (Acylierung) wird üblicherweise bei Normaldruck durchgeführt.

Zur Durchführung der 2. Stufe des erfindungsgemäßen Verfahrens (Acylierung) setzt man pro Mol 4-(Hydroxymethylpiperidinomethyl)-1,3-dioxolan der Formel (Ia) im allgemeinen 1,0 bis 1,3 Mol, vorzugsweise äquimolare Mengen an Acylierungsmittel, und 1,0 bis 1,3 Mol, vorzugsweise äquimolare Mengen, an Base ein.

Die Aufarbeitung und Isolierung der Endprodukte erfolgt nach üblichen Methoden.

Zur Herstellung von pflanzenverträglichen Säureadditionssalzen der Verbindungen der Formel (I) kommen vorzugsweise folgende Säuren infrage : Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure, Milchsäure, sowie Sulfonsäuren, wie z.B. p-Toluolsulfonsäure und 1,5-Naphthalindisulfonsäure.

Die Säureadditionssalze der Verbindungen der Formel (I) können in einfacher Weise nach üblichen Salzbildungsmethoden , z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. bis II. sowie IV. bis VIII. Nebengruppe infrage, wobei Kupfer, Zink, Mangan, Magnesium, Zinn, Eisen und Nickel beispielhaft genannt seien. Als Anionen der Salze kommen solche in Betracht, die sich vorzugsweise von folgenden Säuren ableiten : Halogenwasserstoffsäuren, wie z.B. die Chlorwasserstoffsäure und die Bromwasserstoffsäure, ferner Phosphorsäure, Salpetersäure und die Schwefelsäure.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet , so z.B. als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes und Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemäßen Wirkstoffe mit besonders gutem Erfolg zur Bekämpfung von Getreidekrankheiten, wie beispielsweise gegen den Erreger des Gerstenmehltaus (Erysiphe graminis) oder gegen den Erreger der Blattfleckenkrankheit der Gerste (Pyrenophora teres), von Reiskrankheiten, wie beispielsweise gegen den Erreger der Reisfleckenkrankheit (Pyricularia

oryzae) oder gegen den Erreger Pellicularia sasakii oder von Gemüsekrankheiten,wie beispielsweise gegen den Erreger der Tomatenbraunfäule (Phytophthora infestans) eingesetzt werden. Dabei besitzen die erfindungsgemäßen Wirkstoffe sowohl protektive als auch systemische Wirksamkeit.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und /oder Dispergiermitteln und/oder schaumerzeugenden Mitteln. Im Falle der Benutzung von Wasser als Streckmittel können z.B. auch organische Lösungsmittel als Hilfs lösungsmittel verwendet werden. Als flüssige Lösungsmittel kommen im wesentlichen infrage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylacetat, sowie natürliche Phospholipide, wie Kephaline und Lecithine, oder synthetische Phospholipide. Weitere Additive können mineralische oder vegetalische Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

0133950

Das erfindungsgemäße Verfahren soll durch die folgenden Herstellungsbeispiele erläutert werden:

Herstellungsbeispiele

Beispiel 1 :

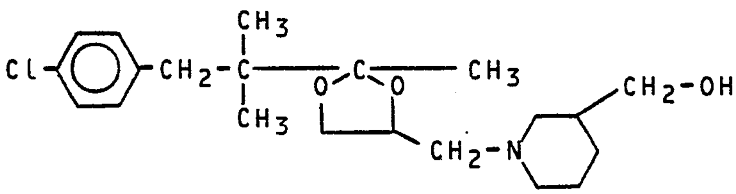

Man erhitzt 30,3 g (0,1 Mol) 4-Chlormethyl-2-[1-(4-chlorphenyl)-2-methyl-prop-2-yl]-2-methyl-1,3-dioxolan, 23 g (0,2 Mol) 3-Hydroxymethylpiperidin und 0,1 g Kaliumjodid in 100 ml Ethanol 15 Stunden lang unter Druck (2 bar) auf 150°C. Zur Aufarbeitung entfernt man das Lösungsmittel im Vakuum, nimmt den Rückstand in 100 ml Methylenchlorid auf, wäscht zweimal mit je 100 ml Wasser, trocknet über Natriumsulfat und entfernt wiederum das Lösungsmittel im Vakuum.

Man erhält 38 g (100 % der Theorie) an 2-[1-(4-Chlorphenyl)-2-methyl-prop-2-yl]-4-[(3-hydroxymethylpiperidin-1-yl)-methyl]-2-methyl-1,3-dioxolan vom Brechungsindex $n_D^{20}$ 1,5226.

Beispiel 2 :

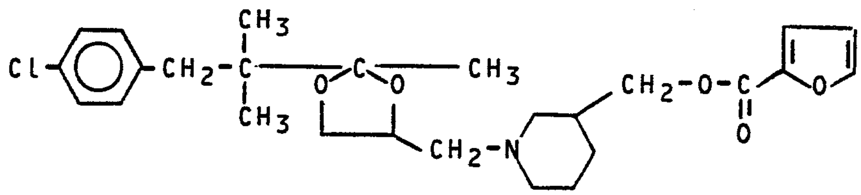

6,9 g (0,018 Mol) 2-[1-(4-Chlorphenyl)-2-methyl-prop-2-yl]-4-[(3-hydroxymethylpiperidin-1-yl)-methyl]-2-methyl-1,3-dioxolan in 70 ml Dichlormethan werden bei Raumtemperatur unter Rühren mit 2,4 g (0,0181 Mol) Furan-2-carbonsäurechlorid versetzt und weitere 2 Stunden gerührt. Zur Aufarbeitung setzt man 50 ml gesättigte Natriumhydrogencarbonatlösungzu, trennt die organische Phase ab, extrahiert die wässrige Phase noch zweimal mit je 50 ml Dichlormethan, trocknet die vereinigten Dichlormethanphasen über Natriumsulfat und entfernt das Lösungsmittel im Vakuum.

Man erhält 6 g (70 % der Theorie) an 2-[1-(4-Chlorphenyl)-2-methyl-prop-2-yl]-4-[3-(furyl-2-carbonyloxymethyl)-piperidin-1-yl-methyl]-2-methyl-1,3-dioxolan als Oel.

$^1H$-NMR ($CDCl_3$) : $\delta$ = 4,17 ppm (d, 2H) entspricht der $-CH_2-O-\underset{\underset{O}{\|}}{C}-$Gruppe.

Herstellung des Ausgangsproduktes :

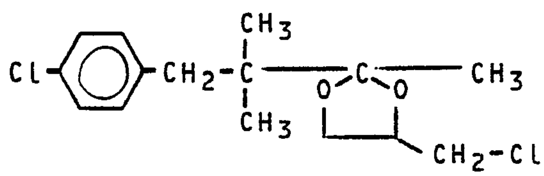

60 g (0,285 Mol) 4-(4-Chlorphenyl)-3,3-dimethylbutan-2-on, 63 g (0,57 Mol) 3-Chlorpropan-1,2-diol und 5,4 g (0,0285 Mol) p-Toluolsulfonsäure werden in einem Gemisch aus 500 ml Toluol und 100 ml n-Butanol 15 Stunden lang über einem Wasserabscheider am Rückfluß erhitzt. Anschließend wird das Lösungsmittel im Wasserstrahlvakuum abdestilliert und der Rückstand im Hochvakuum feindestilliert.

Nach zweimaliger Destillation erhält man 69 g (80 % der Theorie) an 4-Chlormethyl-2-[1-(4-chlorphenyl)-2-methyl-prop-2-yl]-2-methyl-1,3-dioxolan vom Siedepunkt 155°C bei 0,267 mbar als cis-trans-Gemisch.

In entsprechender Weise und gemäß den allgemeinen Herstellungsangaben erhält man die folgenden Verbindungen der allgemeinen Formel (I) :

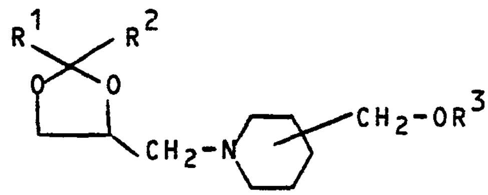

(I)

| Beisp. Nr. | $R^1$ | $R^2$ | 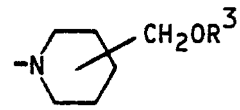 | Brechungsindex ($n_D^{20}$) |
|---|---|---|---|---|
| 3 | 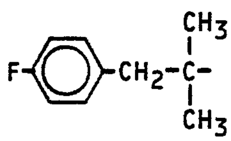 | $CH_3$ | 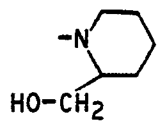 | 1,5173 |
| 4 | 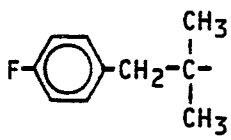 | $CH_3$ | 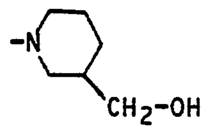 | 1,5123 |
| 5 | 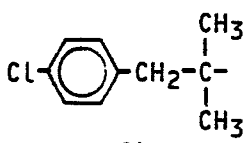 | $CH_3$ | 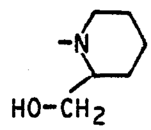 | 1,5269 |
| 6 | 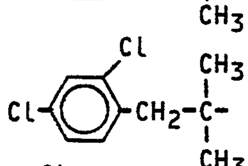 | $CH_3$ | 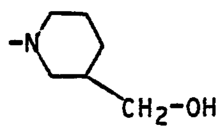 | 1,5308 |
| 7 | 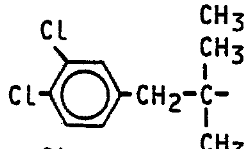 | $CH_3$ | 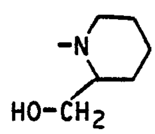 | 1,5361 |
| 8 | 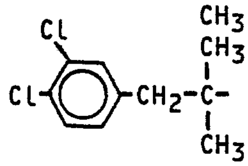 | $CH_3$ | 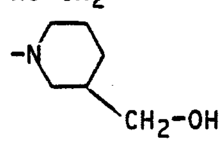 | 1,5362 |
| 9 | 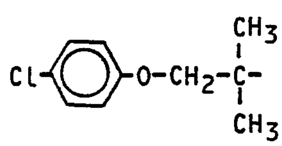 | $CH_3$ | 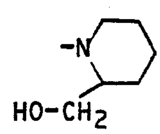 | 1,5249 |

(Fortsetzung)

| Beisp. Nr. | $R^1$ | $R^2$ | 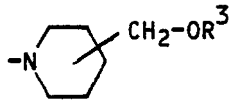 | Brechungsindex ($n_D^{20}$) |
|---|---|---|---|---|
| 10 | 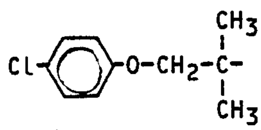 | $CH_3$ | 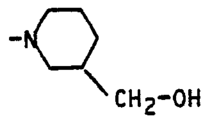 | 1,5196 |
| 11 | 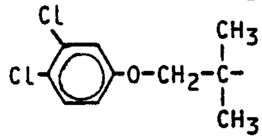 | $CH_3$ | 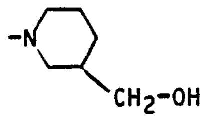 | 1,5114 |
| 12 | 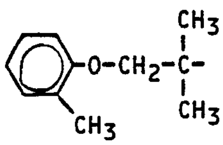 | $CH_3$ | 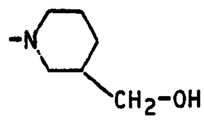 | Oel |
| 13 | 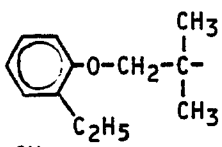 | $CH_3$ | 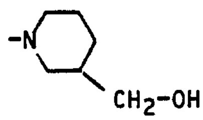 | 1,5041 |
| 14 | 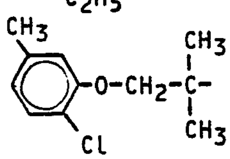 | $CH_3$ | 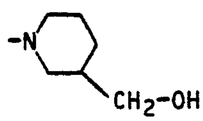 | 1,5112 |
| 15 | 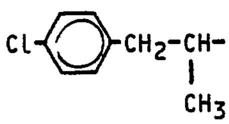 | $CH_3$ | 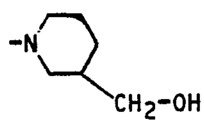 | 1,5273 |
| 16 | 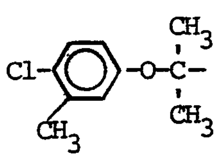 | $CH_3$ | 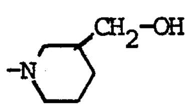 | Kp 250°C/0,1 mbar |
| 17 | 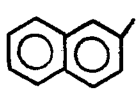 | $CH_3$ | 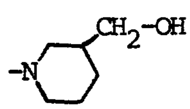 | Kp>250°C/0,1 mbar |

(Fortsetzung)

| Beisp. Nr. | $R^1$ | $R^2$ | 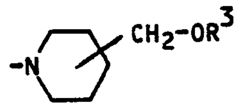 | Brechungsindex ($n_D^{20}$) |
|---|---|---|---|---|
| 18 | 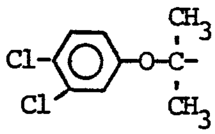 | $CH_3$ | 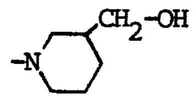 | Kp 250°C/0,1 mbar |
| 19 | 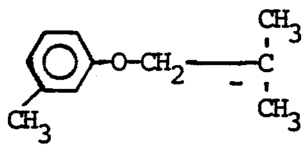 | $CH_3$ | 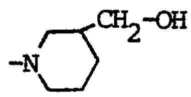 | Kp~200°C/0,1mbar |
| 20 | 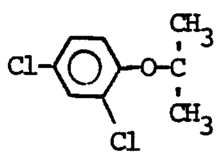 | $CH_3$ | 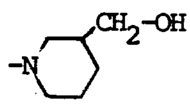 | Kp 250°C/0,1 mbar |
| 21 | 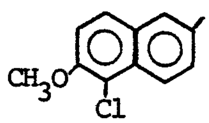 | $CH_3$ | 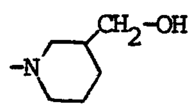 | Oel |
| 22 | 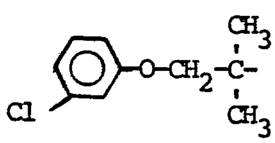 | $CH_3$ | 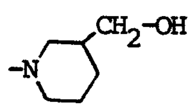 | Kp~200°C/0,1 mbar |
| 23 | 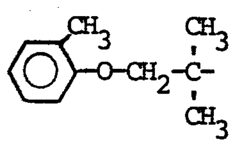 | $CH_3$ | 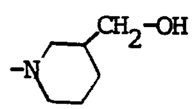 | Oel |

(Fortsetzung)

| Beisp. Nr. | $R^1$ | $R^2$ | 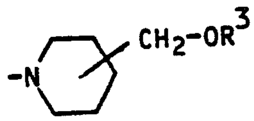 | Brechungsindex ($n_D^{20}$) |
|---|---|---|---|---|
| 24 | 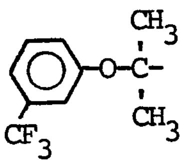 | $CH_3$ | 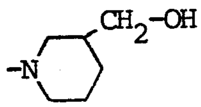 | 1,4810 |
| 25 | 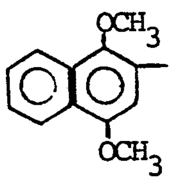 | $CH_3$ | 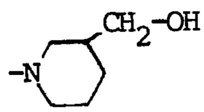 | Oel |
| 26 | 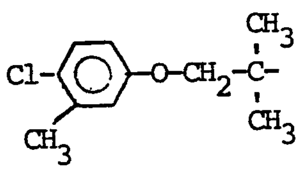 | $CH_3$ | 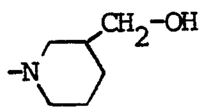 | Oel |

Anwendungsbeispiele :

In den folgenden Anwendungsbeispielen werden die nachstehend aufgeführten Verbindungen als Vergleichssubstanzen verwendet :

(A) 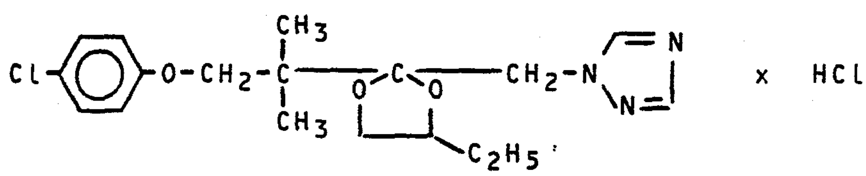

2-[1-(4-Chlorphenoxy)-2-methyl-prop-2-yl]-4-ethyl-2-(1,2,4-triazol-1-yl-methyl)-1,3-dioxolan Hydrochlorid
und

(B) 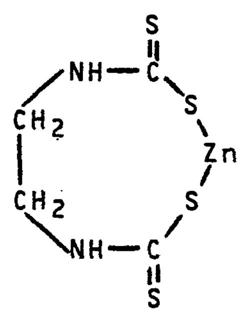

Zink-ethylen-1,2-bis-(dithiocarbamat)

Beispiel A

Phytophthora-Test (Tomate) /protektiv

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit bei ca. 20°C aufgestellt.

3 Tage nach der Inokulation erfolgt die Auswertung.

Eine deutliche Ueberlegenheit in der Wirksamkeit gegenüber dem Stand der Technik zeigt in diesem Test z.B. die Verbindung gemäß Herstellungsbeispiel : 1.

Beispiel B

Erysiphe-Test (Gerste) / protektiv

Lösungsmittel: 100 Gewichtsteile Dimethylformamid
Emulgator: 0,25 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 1, 3, 4 und 10.

Beispiel C

Pyricularia-Test (Reis) / systemisch

Lösungsmittel: 12,5 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: 1, 4 und 10.

Patentansprüche

1. Substituierte 4-Piperidinomethyl-1,3-dioxolane der allgemeinen Formel (I)

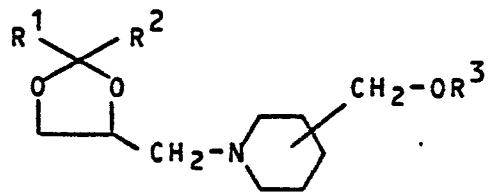

(I)

in welcher

$R^1$ für Tetrahydronaphthyl, Decahydronaphthyl oder für gegebenenfalls substituiertes Naphthyl, für gegebenenfalls substituiertes Cycloalkyl und Cycloalkenyl, für gegebenenfalls substituiertes Phenyl oder für durch - jeweils gegebenenfalls substituiertes-Phenyl, Phenoxy, Phenylthio, Cyclohexyl, Cyclohexyloxy oder Cyclohexylthio substituiertes Alkyl steht,

$R^2$ für Wasserstoff oder Methyl steht und

$R^3$ für Wasserstoff oder einen Acylrest steht

sowie deren pflanzenverträgliche Säureadditionssalze und Metallsalzkomplexe.

2. Substituierte 4-Piperidinomethyl-1,3-dioxolane der Formel (I) nach Anspruch 1, in welcher

$R^1$ für Tetrahydronaphthyl, Decahydronaphthyl oder für gegebenenfalls einfach bis siebenfach, gleich oder verschieden substituiertes Naphthyl steht, wobei als Substituenten in Frage kommen: Hydroxy, Halogen, jeweils geradkettiges oder verzweigtes Alkyl, Alkoxy, Alkenyloxy, Alkinyloxy oder Alkanoyloxy mit jeweils bis zu 4 Kohlenstoffatomen in den Alkylteilen, weiterhin für jeweils gegebenenfalls einfach oder mehrfach, gleich oder verschieden durch geradkettiges oder verzweigtes Alkyl mit bis zu 4 Kohlenstoffatomen substituiertes Cycloalkyl oder Cycloalkenyl mit jeweils 3 bis 7 Kohlenstoffatomen steht, für gegebenenfalls einfach oder mehrfach, gleich oder verschieden substituiertes Phenyl steht, oder für im Phenylkern gegebenenfalls einfach oder mehrfach, gleich oder verschieden substituiertes Phenylalkyl, Phenoxyalkyl oder Phenylthioalkyl mit jeweils bis zu 6 Kohlenstoffatomen im geradkettigen oder verzweigten Alkylteil steht, wobei als Phenylsubstituenten jeweils in Frage kommen: Hydroxy, Halogen, Cyano, Nitro, jeweils geradkettiges oder verzweigtes Alkyl, Alkoxy, Alkenyloxy, Alkinyloxy und Alkylthio mit jeweils bis zu 4 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, Cycloalkyl mit 5 bis 7 Kohlenstoffatomen, geradkettiges oder verzweigtes Alkoxycarbonyl bzw.

Alkanoyloxy mit jeweils bis zu 4 Kohlenstoffatomen im Alkylteil, gegebenenfalls durch Halogen, oder durch geradkettiges oder verzweigtes Alkyl mit bis zu 4 Kohlenstoffatomen substituiertes Phenyl oder Phenoxy, sowie der Rest R-O-N=CH-, wobei R jeweils für geradkettiges oder verzweigtes Alkyl, Alkenyl oder Alkinyl mit bis zu 4 Kohlenstoffatomen steht; außerdem für jeweils im Cyclohexylteil gegebenenfalls einfach oder mehrfach, gleich oder verschieden durch geradkettiges oder verzweigte Alkyl mit bis zu 4 Kohlenstoffatomen substituiertes Cyclohexylalkyl, Cyclohexyoxyalkyl oder Cyclohexylthioalkyl mit jeweils bis zu 6 Kohlenstoffatomen in den geradkettigen oder verzweigten Alkylteilen steht,

$R^2$ für Wasserstoff oder Methyl steht und

$R^3$ für Wasserstoff oder für einen Rest der Formel

$$R^4-\underset{\overset{\|}{O}}{C}-$$

steht, wobei

$R^4$ für geradkettiges oder verzweigtes Alkyl, Alkoxy oder Alkylamino bzw. Dialkylamino mit jeweils 1 bis 6 Kohlenstoffatomen in den einzelnen Alkylteilen, für geradkettiges oder verzweigtes Alkoxyalkyl mit jeweils 1 bis 6

Kohlenstoffatomen in beiden Alkylteilen, für geradkettiges oder verzweigtes Halogenalkyl mit bis zu 6 Kohlenstoffatomen und bis zu 5 gleichen oder verschiedenen Halogenatomen oder für den Furylrest steht.

3. Substituierte 4-Piperidinomethyl-1,3-dioxolane der Formel (I) nach Anspruch 1, in welcher

$R^1$ für Tetrahydronaphthyl, Decahydronaphthyl oder für gegebenenfalls einfach bis dreifach, gleich oder verschieden substituiertes Naphthyl steht, wobei als Substituenten in Frage kommen: Fluor, Chlor, Brom, Iod, Hydroxy, Methyl, Ethyl, Methoxy, Ethoxy, Allyloxy, Propargyloxy und Acetyloxy oder weiterhin für gegebenenfalls einfach bis dreifach, gleich oder verschieden durch Methyl oder Ethyl substituiertes Cycloalkyl oder Cycloalkenyl mit 5 bis 7 Kohlenstoffatomen, für gegebenenfalls einfach bis dreifach, gleich oder verschieden substituiertes Phenyl oder für einen im Phenylkern gegebenenfalls einfach bis dreifach, gleich oder verschieden substituierten Rest der Formel

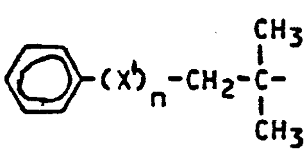 ; 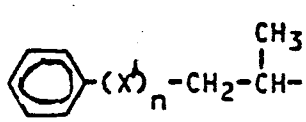 ; 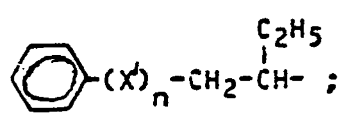 ;

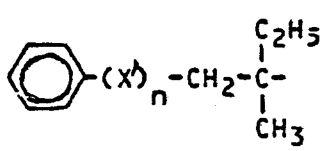 oder 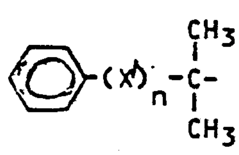 steht,

wobei als Phenylsubstituenten jeweils in Frage kommen: Hydroxy, Fluor, Chlor, Brom, Iod, Cyano, Nitro, Methyl, Methoxy, Methylthio, Ethyl, Ethoxy, Ethylthio, n- und i-Propyl, Isopropyloxy, n-, i-, s- und t-Butyl, Allyloxy, Propargyloxy, Trifluormethyl, Trifluormethoxy, Trifluormethylthio, Cyclohexyl, Methoxycarbonyl, Ethoxycarbonyl, Acetyloxy, gegebenenfalls durch Fluor, Chlor oder Methyl substituiertes Phenyl oder Phenoxy, sowie der Rest R-O-N=CH-, wobei R jeweils für Methyl, Ethyl, n- und i-Propyl, n-, i-, s- und t-Butyl, Allyl und Propargyl steht; außerdem steht $R^1$ für einen im Cyclohexylteil gegebenenfalls einfach bis dreifach, gleich oder verschieden durch Methyl, Ethyl oder Isopropyl substituierten Rest der Formel

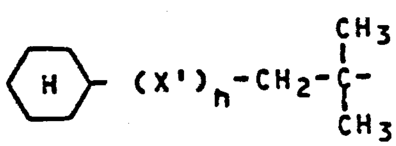 ; 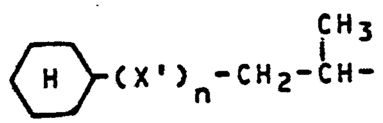 ;

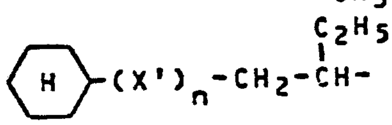 ; 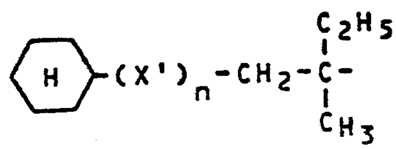 oder

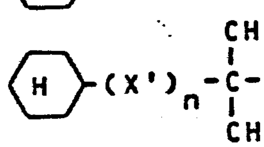

wobei

X' jeweils für Sauerstoff oder Schwefel und

n für 0 oder 1 steht,

$R^2$ für Methyl steht und

$R^3$ für Wasserstoff oder für einen Rest der Formel

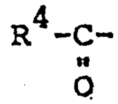

steht, wobei

$R^4$ für Methyl, Ethyl, n- und i-Propyl, Methoxymethyl, Chlormethyl, Dichlormethyl, Trichlormethyl, Methoxy, Ethoxy, Methylamino, Ethylamino, Dimethylamino, Diethylamino oder für den Furylrest steht.

4. Verfahren zur Herstellung von substituierten 4-Piperidinomethyl-1,3-dioxolanen der allgemeinen Formel (I)

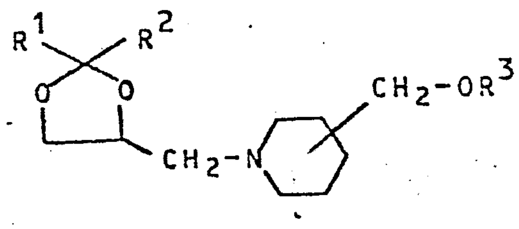

(I)

in welcher

$R^1$ für Tetrahydronaphthyl, Decahydronaphthyl oder für gegebenenfalls substituiertes Naphthyl, für gegebenenfalls substituiertes Cycloalkyl und Cycloalkenyl, für gegebenenfalls substituiertes Phenyl oder für durch - jeweils gegebenenfalls substituiertes -

Phenyl, Phenoxy, Phenylthio, Cyclohexyl, Cyclohexyloxy oder Cyclohexylthio substituiertes Alkyl steht,

$R^2$ für Wasserstoff oder Methyl steht und

$R^3$ für Wasserstoff oder einen Acylrest steht,

sowie deren pflanzenverträgliche Säureadditionssalze und Metallsalzkomplexe, dadurch gekennzeichnet, daß man in 4-Stellung substituierte Dioxolane der allgemeinen Formel (II)

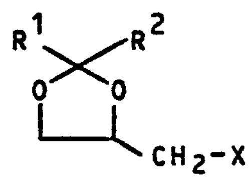

(II)

in welcher

$R^1$ und $R^2$ die oben angegebene Bedeutung haben und

X für Halogen oder für gegebenenfalls substituiertes Alkylsulfonyloxy oder Arylsulfonyloxy steht,

mit einem Hydroxymethylpiperidin der allgemeinen Formel (III)

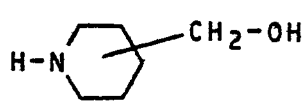

(III)

gegebenenfalls in Gegenwart eines Verdünnungsmittels, gegebenenfalls in Gegenwart eines Katalysators und gegebenenfalls in Gegenwart eines Säurebindemittels umsetzt und gegebenenfalls in einer 2. Stufe die so erhaltenen erfindungsgemäßen 4-(Hydroxymethylpiperidinomethyl)-1,3-dioxolane der allgemeinen Formel (Ia)

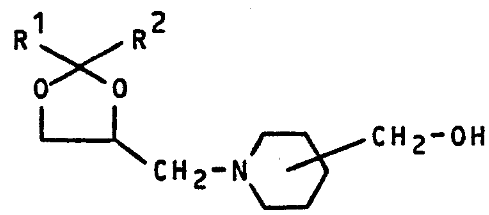

(Ia)

in welcher

$R^1$ und $R^2$ die oben angegebene Bedeutungen haben,

gegebenenfalls in Gegenwart eines Verdünnungsmittels und gegebenenfalls in Gegenwart eines Säurebindemittels mit einem Acylierungsmittel acyliert und gegebenenfalls anschließend eine Säure oder ein Metallsalz addiert.

5. Verfahren nach Anspruch 4, gekennzeichnet durch die Verwendung von Acylhalogeniden oder Anhydriden der Formel

$$R^4\text{-CO-A}$$

in welcher

$R^4$ für geradkettiges oder verzweigtes Alkyl,

Alkoxy oder Alkylamino bzw. Dialkylamino mit jeweils 1 bis 6 Kohlenstoffatomen in den einzelnen Alkylteilen, für geradkettiges oder verzweigtes Alkoxyalkyl mit jeweils 1 bis 6 Kohlenstoffatomen in beiden Alkylteilen, für geradkettiges oder verzweigtes Halogenalkyl mit bis zu 6 Kohlenstoffatomen und bis zu 5 gleichen oder verschiedenen Halogenatomen oder für den Furylrest steht und

A für Halogen oder für den Rest $R^4$-CO-O- steht, worin $R^4$ die oben gegebene Bedeutung hat oder von Isocyanaten der Formel

$$R^5\text{-N=C-O}$$

in welcher

$R^5$ für Alkyl steht, als Acylierungsmittel.

6. Schädlingsbekämpfungsmittel, gekennzeichnet durch einen Gehalt an mindestens einem substituierten 4-Piperidinomethyl-1,3-dioxolan der Formel (I) nach Ansprüchen 1 und 4.

7. Verwendung von substituierten 4-Piperidinomethyl-1,4-dioxolan der Formel (I) nach Ansprüchen 1 und 4 zur Bekämpfung von Schädlingen.

8. Verfahren zur Bekämpfung von Schädlingen, dadurch gekennzeichnet, daß man substituierte 4-Piperidinomethyl-1,3-dioxolane der Formel (I) nach Ansprüchen 1 und 4 auf Schädlinge und/oder ihren Lebensraum einwirken läßt.

9. Verfahren zur Herstellung von Schädlingsbekämpfungsmitteln, dadurch gekennzeichnet, daß man substituierte 4-Piperidinomethyl-1,3-dioxolane der Formel (I) nach Ansprüchen 1 und 4 mit Streckmitteln und/oder oberflächenaktiven Mitteln vermischt.

Europäisches Patentamt

# EUROPÄISCHER RECHERCHENBERICHT

0133950 Nummer der Anmeldung

EP 84108665.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,P | EP - A2 - 0 094 167 (FBC LIMITED)<br>* Formel I *<br>-- | 1 | C 07 D 405/06<br>A 01 N 43/40/<br>(C 07 D 405/06<br>C 07 D 211/22<br>C 07 D 317/28) |
| A | GB - A - 2.095 236 (ICI)<br>* Formel I *<br>-- | 1 | |
| A | EP - A1 - 0 056 461 (BASF)<br>* Zusammenfassung *<br>-- | 1 | |
| A | CHEMICAL ABSTRACTS, Vol. 90, No. 9, 26. Februar 1979, Columbus, Ohio, USA<br>WOLINSKI, JERZY; CZERWINSKA, ANNA " Search for anticholinergic compounds. VII. Synthesis of 2-aminomethyl-,4-aminomethyl-, and 2,4-bis(aminomethyl)-1,3-dioxolanes"<br>Seite 508, Spalte 1, Zusammenfassung Nr. 72 094q<br>& Acta Pol. Pharm. 1978, 35(3), 265-72<br>-- | 1 | RECHERCHIERTE SACHGEBIETE (Int. Cl.4)<br>C 07 D 405/00 |

Der vorliegende Recherchenbericht wurde für alle Patentansprüche erstellt.

| Recherchenort | Abschlußdatum der Recherche | Prüfer |
|---|---|---|
| WIEN | 05-10-1984 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

0133950 Nummer der Anmeldung

EP 84108665.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEMICAL ABSTRACTS, Vol. 95, No. 14, 5. Oktober 1981, Columbus, Ohio, USA<br>KOGAN, R.M.; SIROTKINA, E.E. (TOMSK POLYTECHNIC INSTITUTE) "2-Alkyl-4-(N-carbazolymethyl)dioxolanes as additives to photosensitizers for poly-N-vinylcarbazole"<br>Seite 39, Spalte 2, Zusammenfassung Nr. 116 500d<br>& U.S.S.R. 825,529 From Otkrytiya, Izobret., Prom. Obraztsy, Tovarnye Znaki 1981, (16), 99-100<br>---- | 1 | |

RECHERCHIERTE SACHGEBIETE (Int. Cl.4)

Der vorliegende Recherchenbericht wurde für alle Patentansprüche erstellt.

| Recherchenort | Abschlußdatum der Recherche | Prüfer |
|---|---|---|
| WIEN | 05-10-1984 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82